# EUROPEAN PATENT APPLICATION

(11) **EP 2 060 279 A1**
(43) Date of publication of application: **20.05.2009**
(21) Application number: 08169070.3
(22) Date of filing: 13.11.2008
(51) Int. Cl.: A61L 9/01, A61L 9/014, A61L 9/12

(54) **Odour removal**

(30) Priority: 14.11.2007 GB 0722336
(71) Applicant: Horsley, George, Whitchurch-on Thames, South Oxon RG8 7EW (GB)
(72) Inventor: Horsley, George, Whitchurch-on Thames, South Oxon RG8 7EW (GB)
(74) Representative: Knowles, James Atherton

(57) **Abstract**

A method is described for reducing or removing an unwanted odour in a vehicle lavatory. The method comprises providing an odour-sorbing article comprising an odour-sorbing material, placing the odour-sorbing article in the lavatory. The lavatory will have a volume of 1m³ to 30m³ and the unwanted odour will be a mammalian body odour, a faecal odour and/or a urinary odour. Also described are articles for use in the method. The preferred odour-sorbing material is activated carbon.

## Description

The present invention relates to methods for reducing or removing unwanted odours in lavatories, in particular lavatories in vehicles. The invention also relates to articles for use in such methods, to holders for holding such articles and to lavatories to which the method is applied.

Unwanted odours in public spaces are recognised as a problem. A number of methods have been developed in order to mask or remove such unwanted odour. Previous solutions to such problems usually involve masking the unwanted odour with scented chemicals.

The problem with unwanted odour in lavatories is a common problem, which is especially difficult in the case of lavatories in vehicles (e.g. in bus, coach, train or aircraft lavatories, in particular in commercial aircraft such as passenger aircraft) because of the restricted space in the lavatory and the ways in which air flows in a vehicle. Vehicle lavatories (particularly in trains, buses, coaches or passenger aircraft) may also be used by a significant number of people in a relatively short space of time.

Mitigation of unwanted odours in lavatories is important in order to maintain a pleasant environment. However, there are difficulties in achieving adequate odour removal or masking because of restrictions on weight, space, complexity of systems and use of chemicals in vehicles. Thus, the usual ways of masking unwanted odours using scented chemicals can suffer from disadvantages.

The present invention aims to address the problems discussed above and to provide a method for reducing or removing unwanted odours in lavatories.

The present invention accordingly provides, in a first aspect, a method for reducing or removing an unwanted odour in a vehicle lavatory, the method comprising, providing an odour-sorbing article comprising an odour-sorbing material, and placing the odour-sorbing article in the vehicle lavatory in the presence of the unwanted odour for a period sufficient to reduce or remove the unwanted odour, wherein the lavatory has a volume of 1m³ to 30m³ and wherein the unwanted odour is a mammalian bodily odour, a faecal odour and/or a urinary odour.

Preferably the method is a passive method (i.e. does not involve intentionally forced air-flow over or through the article other than that already present in the space).

Usually the lavatory in which the method is intended to be used will have a volume of between 2m³ to 30m³, 5m³ to 25m³, or 5m³ to 20m³. A plurality of odour-sorbing articles may be used in order to effectively reduce or remove the unwanted odour. Of course, if particularly rapid reduction or removal of the unwanted odour is necessary or if the concentration of odour in the space is particularly great, then more than one odour-sorbing article may be used.

The method is applicable where the unwanted odour is a mammalian body odour, usually a human body odour, a faecal odour and/or urinary odour. Faecal odours typically have a large number of volatile components, including one or more of skatole (3-methylindole), di-isopropyl sulphide, α-pinene, dimethyl disulphide, dimethyl trisulphide, 2-methylthiophene, or 2-pentylfuran. Skatole, dimethyl disulphide and dimethyl trisulphide are particularly common components of faecal odour.

Usually, the ratio of the volume of the lavatory to the volume of the odour-sorbing article(s) will be between 50 and 1 x 10⁷, preferably 1 x 10³ to 1 x 10⁷, more preferably 1 x 10⁴ to 1 x 10⁷, and most preferably 3 x 10⁴ to 5 x 10⁶. As discussed above, if the unwanted odour is particularly concentrated or removal or reduction of the unwanted odour is particularly difficult, then the ratio of the volume of the lavatory to the volume of the odour-sorbing article would be lowered by using, for example, more than one (i.e. a plurality) of odour-sorbing articles.

In a second aspect, the present invention provides an odour-sorbing article for use in a method as claimed in relation to the first aspect. Typically, the article will have a volume in the range of 500cm³ to 5cm³, 300cm³ to 5cm³, or 200cm³ to 5cm³ preferably 100cm³ to 5cm³. The dimensions of the article will usually be less than 50cm, 25cm or preferably 10cm in its largest dimension. More preferably, the article would be less than 8cm in its largest dimension. The usual size of the article according to this aspect of the invention would be a disk of about 5-10cm diameter and less than 6cm thickness, preferably less than 4cm thickness and more preferably less than 2cm thickness.

The odour-sorbing article will usually comprise a matrix loaded with the odour sorbing material. The matrix may be a polymer, e.g. a polymer foam and/or a fibrous material. The matrix may be cellulose (e.g. from wood pulp).

The use of an odour-sorbing article comprising a polymer foam is advantageous because polymer foams can have a significantly enhanced surface area and a porosity which enables an enhanced portion of the area of the foam to be open to the ambient air. The use of an odour-sorbing article comprising polymer foam and an odour-sorbing material can be particularly effective especially when the odour-sorbing material is disbursed evenly throughout the polymer foam (e.g. in an impregnated polymer foam).

The polymer foam of the article may comprise any suitable polymer, for example polyurethane, polyolefin, polyvinylchloride or latex. The preferred polymer comprises polyester fibres and/or cross-linked acrylic polymer.

Most preferably the polymer foam is a non-woven material comprising polyester fibres and cross-linked acrylic polymer.

The odour-sorbing material may comprise any sorbant which absorbs or adsorbs one or more components of the unwanted odour. Such sorption may be by chemisorption, physisorption or a combination of these two processes. The sorbing material may also comprise one or more active compounds to chemically neutralise odour producing volatiles. Preferably, the sorbing material sorbs one or more of the volatile components of the unwanted odour by adsorption. The preferred odour-sorbing material comprises activated carbon. The odour-sorbing material may comprise activated carbon and an additive, for example potassium permanganate.

Thus, a preferred material for producing the odour-sorbing article is a non-woven material comprising polyester fibres and cross-linked acrylic polymer impregnated with activated carbon and potassium permanganate.

Alternatively, the article may comprise cellulose (e.g. cellulose wood pulp), the sorbent may be activated carbon. This is advantageous because cellulose is more biodegradable.

Preferably, the effective surface area of the article is 100-1000m²g⁻¹, preferably 200-800m²/g, more preferably 300-700m²g⁻¹, and most preferably 500-600m²g⁻¹. The loading of sorbent in the article is preferably in the range of 20-90 wt %, more preferably 30-90 wt% and most preferably 40-80wt%.

The article may be any suitable shape, however the preferred shape is round. Preferably the article is a disk. The article may comprise a hole. Thus, in a particular embodiment, the article is (roughly) annular or toroidal.

It is convenient if the article is of a shape adapted for positioning the article in association with either a soap dispenser, a lavatory roll and/or a lavatory roll holder. This is advantageous because the article may then more conveniently be used in a lavatory. The shape may be conveniently adapted to this purpose by providing a hole in the article of a suitable size for placing the article over a soap dispenser or a lavatory roll holder.

In a third aspect of the present invention there is provided a holder adapted to hold an article as discussed above in relation to the second aspect, the holder being such as to allow air to contact the article when the article is held in the holder. Such a holder is convenient because it both improves the aesthetics and provides protection for the odour-sorbing article when in use. The holder will typically have apertures in order to allow ambient air to enter the holder and interact with the odour-sorbing article. The holder may be adapted so that there is a tendency for air to pass through the holder. Such adaptation may be, for example, by ensuring that the holder is rotatable and wherein the holder comprises an air inlet specifically shaped to take in air and direct it onto the odour sorbing article when the holder is manually rotated.

In a fourth aspect of the present invention, there is provided a vehicle lavatory to which the method of the first aspect of the present invention is being applied.

These and other aspects, features and advantages of the invention will more fully be described with reference to the accompanying drawings in which:
Figure 1 is a perspective view of a first odour-sorbing article in accordance with the invention,
Figure 2 is a perspective view of a second odour-sorbing article according to the invention,
Figure 3 is a perspective view of a third odour-sorbing article according to the invention,
Figure 4 are three GC-MS chromatograms produced in association with Example 1, below, in which a) is the control,
   b) is the result using two odour-sorbing articles as illustrated in Figure 1, and
   c) is the result using two odour-sorbing articles as illustrated in Figure 1 wherein the articles are in the reactor and co-axially threaded onto a holder through the holes in the article.

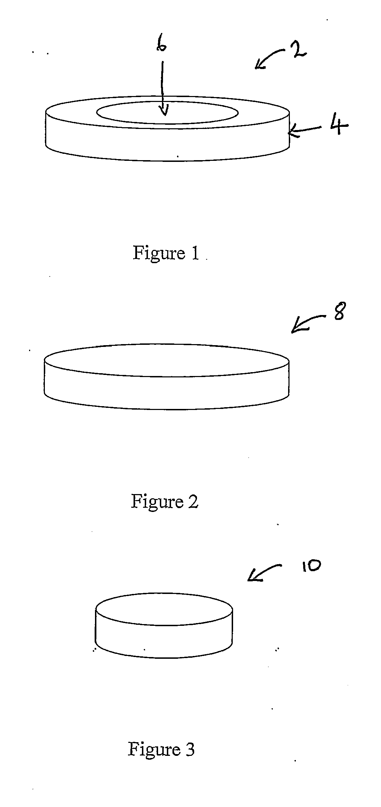

As illustrated in Figure 1, an odour-sorbing article 2 comprises a polymer foam annulus, the polymer foam comprising non-woven polyester fibres and cross-linked acrylic polymer. The polymer foam is impregnated with activated carbon and potassium permanganate. The article 2 contains a hole 6 which is useful in order to associate the article 2 with a lavatory roll holder or a soap dispenser by threading the article 2 on the soap dispenser or lavatory roll holder.

Figure 2 shows an odour-sorbing article 8 which is a disk of the same material as descried in relation to Figure 1.

Figure 3 illustrates a second type of disk article 10 of a smaller dimension.

Articles 2, 8, 10 may be made by cutting out of a sheet of the impregnated polymer material a disk article 8 and further cutting out a hole in the centre of the article 8 to produce the annular article 2 and the small disk article 10.

The present invention will be further illustrated by the Examples discussed below in which an annular article as illustrated in Figure 1 was used to remove or reduce odours in a reactor.

### Example 1

### 1. Selection of Odorous Compounds (Volatiles)

A group of volatile compounds representing different types of chemical compounds (different boiling points, polarity, functional groups etc) was selected and made up into an aqueous cocktail. This cocktail included two compounds, dimethyl disulphide and dimethyl trisulphide, which are known to contribute to the odour of faeces and can be detected by humans at exceptionally low levels (approx 0.0001 ppm). They can also be an indication of microbial action.

Six compounds were dissolved in methanol and made up into an aqueous cocktail at a concentration of 0.02ppm or lower. The odour of the cocktail was strong. The list of compounds used is given in the results section.

### 2. Reaction Vessel

A reaction vessel, capacity approximately 2 litres, was used. A supply of nitrogen (40ml/min) was swept over the aqueous cocktail (contained in a separate flask) into the reaction vessel for 15 minutes, thereby transferring a quantity of the compounds from the headspace of the flask into the reaction vessel. The aqueous cocktail was removed and the reaction vessel sealed and left to equilibrate. After 30 minutes, the contents of the reaction vessel were swept onto a trap which was packed with a material designed to retain and concentrate volatile compounds. The contents of the trap were analysed by GC-MS, (a technique which separates and quantifies the individual components of a mixture of volatiles). This procedure was carried out four times as follows:
1) using an empty reaction vessel (the control);
2) using the reaction vessel with two free annular articles suspended in the middle of the vessel;
3) using the reaction vessel with two annular articles placed over a soap dispensing unit. The soap dispensing unit consists of a solid polymer cylinder of slightly smaller diameter than the hole in the article;
4) a secondary control in which no cocktail was present.

The annular articles are about 6cm in diameter, about 1.5cm thick. The hole has a diameter of about 3.5cm.

### 3. Results

### a) Odour

Sniffing the Vessel: at the end of the experiment it was noticeable that the vessel which did not contain any articles still smelt of the original cocktail but the vessels which had contained articles, in either set-up, did not have any noticeable smell.

### b) Analytical Results

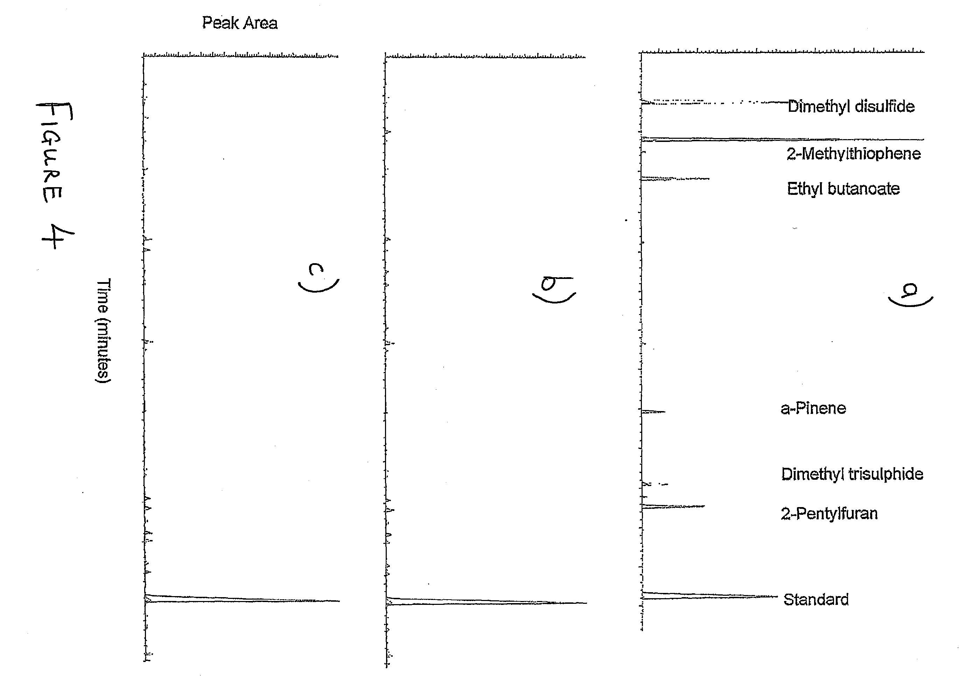

Each run on the GC-MS produces a chromatogram such as those shown in Figure 4. Each peak represents one of the compounds which were contained in the original cocktail and the size of the peak represents the amount present. The last peak in each chromatogram is a standard against which all the other peaks can be measured. It was added to all the traps and is set at a constant value across all the chromatograms.

It is clear from Figure 4 that the articles are extremely effective in reducing the levels of compounds present. The levels of all six compounds have been reduced to trace. These data are shown quantitatively in Table 1. This table shows the percent reduction of each compound for either the two free articles or the two restricted articles.

**Table 1: % Reduction in each of the compounds**

| Compound | Two Free Articles | Restricted Articles |
|---|---|---|
| Di-isopropyl sulphide | 100% | 100% |
| Ethyl butanoate | 100% | 100% |
| α-Pinene | 100% | 100% |
| Dimethyl disulfide | 99.9% | 99.4% |
| Dimethyl trisulphide | 99.8% | 98.8% |
| 2-Methylthiophene | 99.7% | 98.8% |
| 2-Pentylfuran | 98.7% | 96.1% |

When the articles were present, three volatiles were no longer detected by the GC-MS and have been essentially removed from the system. The two faecal-like compounds (dimethyl disulfide and dimethyl trisulphide) were reduced by 99.8% when the articles were free and slightly less using the restricted rings. In a preliminary trial, another faecal-like compound (skatole) was included in the cocktail and it showed a similar drop. These data show a reduction of at ∼99% for all volatiles when the free articles were used and slightly less when the articles were attached to the soap dispensing unit.

### 4. Conclusion

This study presents convincing evidence that the articles, even when attached to the soap dispensing unit, substantially reduce the levels of volatile compounds in the surrounding atmosphere (∼99% reduction).

### Example 2

In Example 2, annular articles as described in Example 1 were used to remove odour from a flooded stairwell.

The basement stairwell (the base of which is 1.05 m by 1.4 m, height 2.33 m) was flooded following a period of heavy rain. The water was removed. No attempt was made to clean the stairwell. Four annular articles were placed in the stairwell. Within 24 hours the unpleasant odour following flooding was much reduced. After 48 hours, the odour was hardly noticeable.

Example 2, therefore, illustrates that the method and article according to the invention are highly effective at removing or reducing odours.

### Example 3

In this Example the odour-sorbing article was an air-laid web material consisting of powdered activated carbon and cellulose wood pulp bound on to a viscose backing with modified acrylonitrile copolymer latex. The carbon surface area was in the range of 900-1100m²/g. The carbon content was about 60% by weight, the effective surface area of the article was about 560m²/g_{.}

A standard vapour test using a model compound trimethyl pentane (TMP) was used and 2 annular articles as described in Example 1 were exposed to TMP. The 2 annular articles adsorbed 0.040g and 0.039g TMP. The standard vapour test was also conducted using cellulose/carbon odour sorbing articles as described above of the same size and shape as the articles of Example 1. One of the annuli adsorbed 0.22g TMP and the other 0.21g TMP showing that these articles are likely to have enhanced adsorption properties.

## Claims

1. A method for reducing or removing an unwanted odour in a vehicle lavatory, the method comprising,
providing an odour-sorbing article comprising an odour-sorbing material, and
placing the odour-sorbing article in the lavatory in the presence of the unwanted odour for a period sufficient to reduce or remove the unwanted odour, wherein the lavatory has a volume of 1m³ to 30m³ and wherein the unwanted odour is a mammalian bodily odour, a faecal odour and/or a urinary odour.

2. A method as claimed in claim 1, wherein the unwanted odour is at least partly due to the odour of one or more of skatole, di-isopropyl sulphide, α-pinene, dimethyl disulphide, dimethyl trisulphide, 2-methylthiophene or 2-pentylfuran.

3. A method as claimed in either claim 1 or claim 2, wherein the ratio of the volume of the lavatory to the volume of the odour-sorbing article is between 50 and 1 x 10⁷.

4. An odour-sorbing article for use in a method as claimed in any one of claims 1 to 3.

5. An article as claimed in claim 4, wherein the article has a volume in the range 5cm³ to 100cm³.

6. An article as claimed in either claim 4 or 5, comprising a matrix.

7. An article as claimed in claim 6, wherein the matrix comprises a polymer foam comprising a polymer selected from either polyester fibres, cross-linked acrylic polymer or both.

8. An article as claimed in claim 7, wherein the polymer foam is a non-woven material comprising polyester fibres and cross-linked acrylic polymer.

9. An article as claimed in claim 6, wherein the matrix comprises cellulose.

10. An article as claimed in any one of claims 4 to 9, wherein the odour-sorbing material comprises activated carbon and/or potassium permanganate.

11. An article as claimed in any one of claims 4 to 10, wherein the effective surface area of the article is 200-800m²g⁻¹.

12. An article as claimed in any one of claims 4 to 11, wherein the article is of a shape adapted for positioning the article in association with either a soap dispenser, a lavatory roll and/or a lavatory roll holder.

13. A holder adapted to hold an article as claimed in any one of claims 4 to 12, the holder being adapted so that air can contact the article when the article is held in the holder.

14. A vehicle lavatory to which the method of any one of claims 1 to 3 is being applied.
